# EUROPEAN PATENT APPLICATION

(11) **EP 3 336 814 A1**
(43) Date of publication of application: **20.06.2018**
(21) Application number: 16835530.3
(22) Date of filing: 10.08.2016
(51) Int. Cl.: G08B 17/00, C07C 19/00

(54) **METHOD OF DETECTING PRE-FIRE SITUATIONS ARISING AS A RESULT OF ELECTRICAL CIRCUIT FAULTS**

(30) Priority: 10.08.2015 RU 2015133305
(71) Applicant: Limited Liability Company "Termoelektrica", Moscow 143026 (RU)
(72) Inventor: LESIV, Aleksey Valeryevich, Dolgoprudny 141707 (RU)
(74) Representative: Chimini, Francesco
(86) International application number: PCT/RU2016/000529
(87) International publication number: WO 2017/026920

(57) **Abstract**

The present invention relates to the field of fire and electric power safety, in particular to methods for detection of pre-fire situations arising from local overheating of electrical equipment, and is intended to prevent fires arising from electrical wiring faults, in particular, faults in wiring devices. The method is based on measuring the content of low-boiling substances in the atmosphere of the protected room, which are enclosed in capsules with an opening temperature in the range of 80-200 ° C, fixed on current-carrying parts, and released into the external environment when these capsules are opened by heating the mentioned parts to the appropriate temperature. The technical result of the claimed solution is to increase the probability of detecting a pre-fire situation at an early stage.

## Description

### Field of the invention

The present invention relates to the field of fire and electric power safety, in particular to methods for detection of pre-fire situations arising from local overheating of electrical equipment.

### Background of the invention

To date, more than 20% of all fires occur due to violations in the operation of the electrical equipment and electrical devices. Most often, ignition occurs in the area of electrical contacts.

One of the most effective methods of fire fighting is the recognition of pre-fire situations. Many systems for detecting such situations are based on monitoring the composition of the gaseous medium, in particular on the analysis of the content of gases released at the initial stage of combustion (smoldering).

Hydrogen (H₂) is the main component of the released gases at the stage of smoldering as a result of the pyrolysis of materials used in construction, such as wood, textiles, synthetic materials. At the initial stage of the fire, during the smoldering process, the hydrogen concentration is 0.001-0.002%. Further the content of aromatic hydrocarbons is increasing against the background of the under-oxidized carbon presence in the form of carbon monoxide (CO)-0,002-0,008% (vol.% in air) [1].

Experiments have demonstrated that the threshold for early fire alarm system in the atmospheric air under normal conditions should be at a level of 0.002% for most gases, including hydrogen and carbon monoxide. At the same time, it is desirable that the system performance be at least 10 s. This conclusion can be considered as a fundamental for the development of a number of warning fire gaseous alarms [1].

However, as indicated above, the concentrations of the gaseous thermal decomposition products formed in the smoldering stage are very small. Because of this, all systems for detecting pre-fire situations, based on the detection of such products in the air, have a number of common shortcomings:

Use of such systems is possible only on objects with a low degree of ventilation.

To detect low concentrations of CO and H₂, high-precision selective methods shall be used. At the same time, the gas sensors can not provide the required selectivity, and devices based on spectrometric measurements have a high cost and are difficult to maintain.

Since the appearance of such small concentrations of combustion products in the air can occur not only as a result of ignition, an increase in the sensitivity of detection systems leads to an increase in the number of false responses. So, for example, immediately after the appearance of the flame, the concentration of carbon dioxide (CO₂) increases to 0.1 %, which on the one hand corresponds to the combustion of 40-50 g of wood or paper in a closed room with a volume of 60 m³, on the other hand it is equivalent to 10 smoked cigarettes. Such level of CO₂ is also achieved as a result of the presence of two people in a room for 1 hour [1].

Since intensive separation of thermal decomposition products begins only at high temperatures (> 250 ° C), i.e. shortly before the appearance of a flame, such systems do not allow identifying dangerous situations at the early stages.

Thus, a method is known for diagnosing a pre-fire situation and preventing a fire, including measuring the intensity of monochromatic radiation emitted by a pulsed source at the frequency of its absorption by thermal destruction products of the identified materials, and generating a control signal for fire alarm when the concentrations of their admissible values are exceeded [2].

The disadvantages of the known method include its low reliability, high probability of false responses, as well as insufficiently early detection of fires, which is caused by the development of a control signal without taking into account the rate of increase in concentration and the assessment of a fire hazard situation with respect to the concentrations of insufficient quantities of controlled gas components.

A method and a device for detecting a pre-fire situation based on the infrared spectroscopy is known. The device comprises an optically coupled source and a radiation receiver coupled to the first amplifier and a processing pattern that includes two radiation receivers, the second and the third amplifier which, together with the first amplifier, are connected to an analog-to-digital converter through the respective blocks of admissible concentrations of fire hazardous components, the output of the converter is connected through the microprocessor and digital-to-analog converter to the alarm unit, while the second output of the microprocessor is connected to the monitor. It is designed to detect the products of thermal decomposition of various organic materials formed under the influence of a non-standard heat source, which can arise, in particular, as a result of sparking or short-circuiting in the electrical commutation equipment. [3].

The disadvantage of the known technical solution is that it reacts to the appearance of gases and smoke accompanying the already started ignition, i.e. it gives a signal directly at the moment of ignition start or after the start of it.

A device for monitoring the parameters of a gaseous medium is known, containing gas sensors, an analog measuring part, a microprocessor module for controlling the operating modes of the sensors, the primary processing of measurement data and their storage, as well as the power supply circuit of the sensor and the device as a whole, characterized by the fact that the electronic circuit of the device integrates software and hardware interface for data transmission and commands over wireless networks, and the algorithm for measuring and transmitting data is optimized for the purpose of an autonomous operation the facility without replacing the battery during the calibration interval. In this case, the device can be used as a pre-alarm detector to control the chemical composition of air, in particular, to determine the content CO and H₂ [4].

The disadvantage of the known device is the possibility of false responses in the detection of pre-fire situations, as well as low operational reliability during the maintenance period due to high sensitivity to interference.

Another known method for diagnosing a pre-fire situation and prevention of a fire, including measuring of informative parameters by a sensor unit: concentrations of gaseous thermodestruction products in air, namely CO, CO₂, NOₓ, HCl, oxidants, fume, as well as temperature, measurement of the signal lag time from each of the sensors using an ignition simulator, determining the derivatives values of the time dependence on the information parameters measured by each sensor , the generation of a control signal for fire alarm start and the possible activation of fire extinguishing means and switching off the power supply as a result of a fire risk analysis based on measured by, at least, two sensors of informative parameters, characterized by the fact that in addition as informative parameters, measure concentrations of H₂, CH₄, NH₃, O₂, Cl₂, H₂S, SO₂, HCOH, C₆H₅OH, reducing agents in the time interval 0.1 -60 s they determine for each dependence of information parameters on time, at least one value of the derivative, determine the modified value of each of the measured informative parameters as a value equal to the product of the derivative value per time corresponding to each lag sensor and produce the control signal when the permissible values are exceeded by the modified values of the informative parameters determined from the measurements of at least two sensors, the time delay of the signal being periodically measured as the value of the time interval between the switching times of the fire simulator and the maximum value of the signal from the sensor [ 5].

The known method is applicable in wide use to a limited extent because of the complexity of measuring the concentrations of gaseous thermodestruction products in air, the inertia of the measurements and the need for expensive equipment.

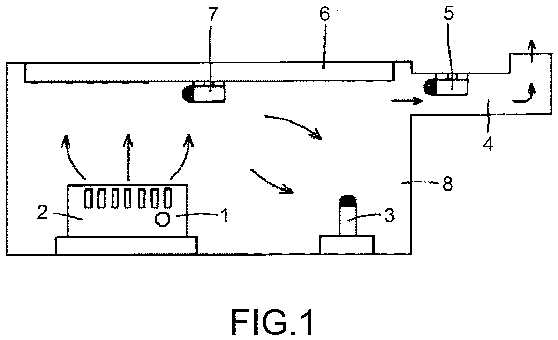

A somewhat different approach to the recognition of pre-fire situations is described in the patent document [6], which discloses a device for the early detection of overheating in hard-to-reach points of electrical and mechanical equipment, which is based on the use of an odorant 1 sealed in a hot-melt composition installed near the heat generating part of the device 2, for which overheating is controlled (see Fig 1). An odor sensor 3 is installed downstream from the gas flow from this odorant (see Fig 1). As an odorant, microcapsules of a hot melt composition containing flavoring agents can be used. Also, flavors mixed with the wax or other fatty acids can be applied. This technical solution is the closest analogue of the present invention.

The drawback of the solution known from [6] is the use of hot melt polymers. When the heat-generating part is heated above the softening or melting temperature of the hot-melt polymer, it may be detached or drained to a part of the electrical equipment, for example, to the insulation of the wiring, the violation of which can lead to a short circuit. In addition, the description of the patent document [6] indicates that the odorant emission from the proposed polymeric compositions occurs due to the melting of the material. This circumstance can be accompanied by unfavorable consequences for the electrical equipment, such as foaming and spraying of the polymeric mass with the evolved gas. Insertion of hot foamed mass, polymer droplets or polymer melt flowing off the sticker to the adjacent contacts, electrical equipment, blowers, sensors, can lead to malfunction or even ignition.

In addition, for the registration of pre-fire situations one of the most significant criteria is the response speed of the system as a whole. For these purposes, the gas shall be released in a significant amount when the critical temperature is reached and quickly distributed in volume. However, pore opening resulting from the melting of the polymer in [6] may be accompanied by the transition of the odorant to a hot melt composition (eg, dissolution) or to create a foam layer. In this case, the evaporation of gas from the surface will proceed slowly and will not lead to a one-time transition of the main amount of gas enclosed in the product into the gas phase.

Moreover, a significant disadvantage of the system is the rate of gas emission during slow heating of the microencapsulated odorant or the odorant mixed with wax or fatty acids. Since the first the layer of material to be melted is the one closest to the source of heat, then the next, and so on, the exit velocity of the odorous substance will be negligible. If there is ventilation, the concentration of the odorous substance may be negligible, so that the sensor will not react.

### Description of the invention

The object of the invention is to increase the probability of detecting a pre-fire situation at an early stage and to minimize the number of false responses.

The essence of the technical solution lies in the fact that to detect pre-fire situations on the heat-sensitive sections of the electric circuit, one or several capsules containing low-boiling matter inside and having an opening temperature in the range of 80-200 ° C are fixed with adhesive or adhesive tape and the content of this light boiling substance in the atmosphere of the protected room is measured by means of a gas sensor connected to a logger that is connected to the signal delivery system. When heated above a certain temperature, the capsules are opened, accompanied by the release of vapors of a light boiling substance, which is detected by a gas sensor.

In contrast to the prototype disclosed in the patent document [6], instead of the plurality of microcapsules contained in the hot melt composition or dispersed in the hot melt aromatizing agents polymers, the offered method uses one or more large capsules. In this case, regardless of the rate of heating, the capsule is opened at a time and a single emission of all or a significant amount of gas occurs. The one-time release of gas contributes to the creation of a high concentration of gas in the volume and the reliable operation of the gas sensor, regardless of air exchange (see Example 6).

Normally opening of the tube (macrocapsule) when heated occurs in the place of secondary filling. Due to the manufacturing features, each tube has a different thickness of the primary shell at the sealing location and its own opening temperature. The latter circumstance causes an important advantage of the proposed invention. Since the opening of the capsules occurs over a relatively wide temperature range, the proposed method, in case of using several capsules, allows the overheating to be recorded multiple times. In other words, if several capsules are heated to a temperature in the opening temperature range, cooled and reheated to a temperature within the opening temperature range (but higher than the first time), then when reheated the capsules that were not opened for the first time will release gas sufficient to record overheating.

The method ensures early detection of pre-fire situations when heating of wires or electrical contacts exceeds the permissible operating parameters (> 100 ° C), but does not yet reach the level at which thermal destruction of materials capable of ignition occurs (> 250 ° C).

### BRIEF DESCRIPTION OF THE DRAWINGS

Fig. 1 shows a known device for early detection of overheating in hard-to-reach points of electrical and mechanical equipment (according to the patent document [6]).
Fig. 2 schematically shows a general view of the alarm device.
Fig. 3 shows the time dependences of the gas concentration (green curve) and the temperature of the heating plate (red curve) in the event of repeated response of the system. 6).

### Detailed description of the invention

The method of the present invention is designed to detect pre-fire situations that arise as a result of local overheating of electrical equipment. The present method is characterized by the following - one or more capsules containing an easy-boiling substance and having an opening temperature in the range of 80-200 ° C are fixed to the heat-prone areas of the electrical circuit with adhesive or adhesive tape and the content of this easy-boiling matter is measured in the atmosphere of protected premises with a gas sensor connected to the recorder, which is connected to the signal delivery system.

Crosslinked (thermosetting) polymers are preferably to be used as the capsule material. In this case, it is possible to avoid the drawbacks associated with melting of the material shall and leakage onto the electrical equipment.

As the light-boiling substance contained in the capsules, freons such as 1,1,1,3,3-pentafluorobutane (chladone 365) and 1,1,1,2,2,4,5,5,5-nonafluorane -4- (trifluoromethyl) pentan-3-one (Novec 1230) or sulfur dioxide can be used. These compounds belong to the 4th danger class, i.e. are not hazardous to humans. During the normal operation of the equipment, freons are not present in the air of the premises under normal circumstances, so they can be detected at minimum concentrations, without fear of false responses. In addition, freons are a class of compounds that can be selectively detected by special sensors at the lowest concentrations (up to 0.001 ppm), which makes the system reliable even when using forced ventilation or protecting electrical equipment in large volumes [7].

As a light-boiling substance, alternatively or additionally, odorants, such as low-molecular-weight mercaptans, dialkyl sulfides, dialkyl disulfides or their solutions, may also be used. The advantage of this solution is that in this case, the detection of overheating will be possible not only with the help of a sensor, but also with the help of a human sense of smell.
a. Odorant may be methyl mercaptan, ethyl mercaptan, n-propyl mercaptan, isopropilmerkaptan, n-butyl, sec-butyl mercaptan, isobutilmerkaptan, tert-butyl mercaptan, amilmerkaptan, isoamilmercaptan, hexylmercaptan, dimethyl, diethyl, diallyl disulfide, allilmetilsulfid, methyl ethyl, diisopropilsulfide, dimethyl disulfide, diethyl disulfide, dipyridyl disulfide , diisopropyldisulfide.

In some embodiments, the odorants are used in a mixture with solvents. The use of solvents allows achieving lower temperatures and narrower temperature ranges of capsule opening.

Odorant solvents include, but are not limited to this list, hydrofluorochlorocarbons, hydrofluorocarbons, fluorocarbons, alkanes, ethers, or mixtures thereof.

Capsules made of polymeric material have the shape of a cylinder with rounded ends. The diameter of the capsules is 1-10 mm, the length is 5-50 mm, the thickness of the polymeric shell is 0.1-1 mm. Capsules are fixed to the current-carrying part by means of glue or adhesive tape.

As the material of the polymeric shell of the capsules, polyurethane, polyurea, polyvinyl acetate, crosslinked gelatin can be used.

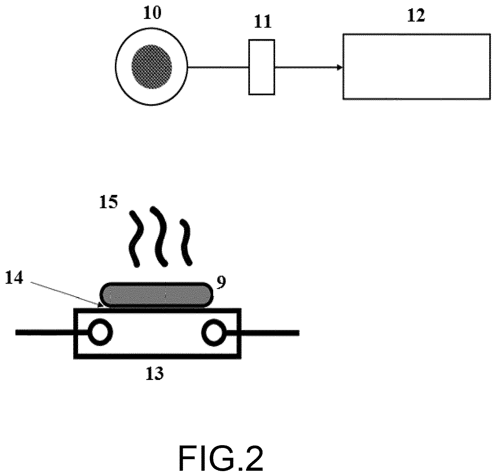

The proposed operations of the method are explained by graphical materials, where Fig. 2 schematically shows a general view of a system implementing the proposed method for detecting a pre-fire situation.

The pre-fire alarm system consists of a capsule 9 with a shell made of polymeric material, a gas sensor 10 connected via a registrar 11 to a signal delivery system 12. The capsule 9 is fixed to the current-carrying part 13 by means of an adhesive layer 14. When heated above a certain temperature, the capsule 9 releases the gas contained therein 15, detected by the gas sensor 10.

Since the gaseous substances released by heating the composite material are not present under normal conditions in the atmosphere, and also because they are released at relatively low temperatures (before the thermal decomposition of the materials from which wires and wiring devices are made), the invention allows to detect potentially fire hazardous situations long before the appearance of smoke or open fire.

Thus, the proposed invention makes it possible to detect pre-fire situations much earlier than existing analogues. The method is easy to implement, does not require the use of expensive equipment or complex methods of processing incoming data.

### Example 1.

The polyamide tube with a diameter of 1 mm and a shell thickness of 0.25 mm is cut into pieces of 70 mm.

The tube is clamped in the holder and is melted at both ends with a hot air stream. Then, the tube is grasped by the ends, the middle is heated up to the beginning of melting and stretched in different directions by 3 mm.

The tube is cut in the middle, vacuum-processed and filled with 1,1,1,3,3-pentafluorobutane. The filled tube is cooled, fixed in the holder and the thin end is sealed with a metal plate.

The resulting capsule is vacuum-processed for 1 hour and checked for leaks in weight loss. Empty capsules are sent for re-melting. The full capsule is heated to 80C, and then again vacuum-processed. If the capsule remains filled, it is used in the method of the present invention.

### Example 2

The capsule is prepared according to the example 1, with the difference that the obtained tube is placed in the center of a casting mold with a size of 2x3x30 mm and filled with epoxy resin.

### Example 3

The capsule is prepared in the same manner as in Example 2, with the difference that the tube is filled with 1,1,1,2,3,3,3-heptafluoropropane.

### Example 4

The capsule is prepared in the same manner as in Example 2, with the difference that the tube is filled with a 2% (by weight) solution of dimethyl sulfide in 1,1,1,2,2,4,5,5,5-nonafluoro-4- (trifluoromethyl) pentane-3-onone.

### Example 5

The capsule is prepared in the same manner as in Example 2, with the difference that a bundle of 10 tubes made according to Example 2 is placed in a casting mold with a size of 20x3x30 mm and filled with a polyester resin. The resulting plate is attached to a double-sided scotch tape.

### Example 6

The response of the invention method upon an instant heating of a single capsule in a 1m³ volume cabinet.

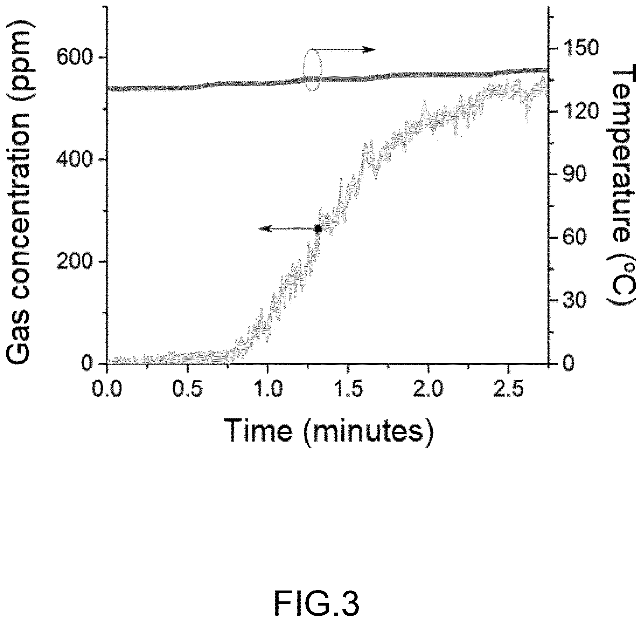

Testing procedure. The capsule made according to Example 1 was glued to the heating plate, the temperature of which was maintained in the range of 130 to 135 ° C. The plate was placed in the geometric center of the cabinet with a volume of 1 m³ (the height of the cabinet was 2.0 m, the width was 1.0 m and the depth was 0.5 m). The temperature of the plate was controlled by a thermocouple fixed between the product of the composite polymeric material and the heating plate. The concentration of the signal gas released by the composite polymeric material was registered with a semiconductor gas sensor SP-42A-00 (manufactured by FIS Inc.) located at the distance of 1 cm from the geometric center of the upper edge of the cabinet. Fig. 3 shows the time dependences of the gas concentration (green curve) and the temperature of the heating plate (red curve).

As it can be seen from Fig. 3, the system response and overheating recording by means of the gas sensor takes place in less than 1 minute.

### Information sources:

1. Electronics: Science, Technology, Business. Issue 4/2001, p. 48.
   Author's certificate of the USSR 1277159, IPC G08B17 / 10, 1985.
   Patent of the Russian Federation No. 2022250, IPC G01N21 / 61, 1994.
4. The patent of the Russian Federation 95849, IPC G01N33 / 00, 2010.
5. The patent of the Russian Federation 2175779, IPC G08B17 / 117, 2001.
6. Patent document JP 6-66648, 1994.
7. A.P.Dolin, A.I. Karapuzikov, Yu.A. Kovalkova, "Efficiency of using a laser leak detector "KARAT" to determine the location and level of development of electrical equipment malfunction", Electro, Nº 6. PP. 25-28 (2009).
8. The patent of the Russian Federation 2403934, IPC A62D1 / 00, 2010.

## Claims

1. The method to detect pre-fire situations is **characterized by** the following - one or more capsules containing an easy-boiling substance and having an opening temperature in the range of 80-200 ° C are fixed to the heat-prone areas of the electrical circuit with adhesive or adhesive tape and the content of this easy-boiling matter is measured in the atmosphere of protected premises with a gas sensor connected to the recorder, which is connected to the signal delivery system.

2. The method to detect pre-fire situations according to claim 1, differing by the fact that it is designed for multiple responses.

3. The method to detect pre-fire situations according to claim 1 or 2, differing by the fact that the material of capsules and glue are thermosetting polymers.

4. The method to detect pre-fire situations as according to claims 1-3, differing by the fact that fluoro-containing halocarbons are used as the light-boiling substance contained within the capsules.

5. The method to detect pre-fire situations according to claim 4, differing by the fact that 1,1,1,3,3-pentafluorobutane (Hladon 365), 1,1,1,2,2,4,5,5, 5-nonafluoro-4-(trifluoromethyl) pentan-3-one (Novec 1230), 1,1,1,2,3,3,3 heptafluoropropane, octafluoropropane, sulfur hexafluoride, pentafluoroethane, octafluorocyclobutane or mixtures thereof are used as easy-boiling substance.

6. The method to detect pre-fire situations according to claims 1-3, differing by the fact that easy-boiling substance contained within the capsules are the odorants selected from the group consisting of methyl mercaptan, ethyl mercaptan, n-propyl mercaptan, isopropyl mercaptan, n-butyl mercaptan, sec-butyl mercaptan, isobutyl mercaptan, tert-butyl mercaptan, amyl mercaptan, isoamyl mercaptan, hexyl mercaptan, dimethyl sulfide, diethyl sulfide, diallyldisulfide, allyl methyl sulfide, methylethylsulfide, diisopropyl sulfide, dimethyl disulfide, diethyldisullfid, dipyridyl disulfide, diisopropildisulfide or solutions thereof.

7. The method to detect pre-fire situations according to claim 6, differing by the fact that the solvent in odorant solutions includes hydrofluorochlorocarbons, hydro fluorocarbons, fluorocarbons, alkanes, ethers or any mixtures thereof.

8. The method to detect pre-fire situations according to claims 1-7, differing by the fact that the material for the capsule shell is polyurethane, polyurea, polyvinyl acetate, crosslinked gelatin, polyamide, polyester, polyvinyl chloride or fluorine-containing polymers, such as vinylidene fluoride or fluoroplastic.
